# EUROPÄISCHE PATENTSCHRIFT

(11) **EP 0 975 575 B1**
(45) Veröffentlichungstag und Bekanntmachung des Hinweises auf die Patenterteilung: **26.06.2002**
(21) Anmeldenummer: 98928192.8
(22) Anmeldetag: 07.04.1998
(51) Int. Cl.: C07C 67/36, C07C 69/606, C07C 69/618, C07C 67/00

(54) **VERFAHREN ZUR HERSTELLUNG VON 2-ALKINSÄUREESTERN**
METHOD FOR PRODUCING 2-ALKYNOIC ACID ESTERS
PROCEDE DE PRODUCTION D'ESTERS D'ACIDE 2-ALCYNIQUE

(30) Priorität: 15.04.1997 DE 19715697
(43) Veröffentlichungstag der Anmeldung: 02.02.2000
(73) Patentinhaber: BASF AKTIENGESELLSCHAFT, 67056 Ludwigshafen (DE)
(72) Erfinder: HENKELMANN, Jochem, D-68165 Mannheim (DE); PREISS, Thomas, D-67065 Ludwigshafen (DE); BÖTTCHER, Arnd, D-67227 Frankenthal (DE); GLEITER, Rolf, D-69120 Heidelberg (DE); DE BACKER, Christel, B-2812 Muizen (BE)
(74) Vertreter: Isenbruck, Günter
(86) Internationale Anmeldenummer: EP9802021
(87) Internationale Veröffentlichungsnummer: WO9846555

(56) Entgegenhaltungen:
- HISASHI YAMAMOTO ET AL.: "Total Synthesis of Celacinnine,Celallocinnine,Celafurine and Celabenzine" JOURNAL OF THE AMERICAN CHEMICAL SOCIETY., Bd. 103, Nr. 20, 7. Oktober 1981, Seiten 6133-6136, XP002075396 DC US
- PATENT ABSTRACTS OF JAPAN vol. 5, no. 168 (C-077), 27. Oktober 1981 & JP 56 097250 A (NIPPON ZEON CO LTD), 5. August 1981 in der Anmeldung erwähnt

## Beschreibung

Die Erfindung betrifft ein Verfahren zur Herstellung von 2-Alkinsäureestern und die Verwendung bestimmter Katalysatoren zu ihrer Herstellung.

2-Alkinsäureester wie Propiolsäureester sind wichtige organische Synthesebausteine und finden unter anderem auf dem Pharmasektor Verwendung. Sie können weiterhin als Fungizide oder Bakterizide eingesetzt werden.

Zu ihrer Darstellung sind zahlreiche Verfahren bekannt.

Aus JP-A 56 097 250 ist die Herstellung von 2-Alkinsäureestern durch Umsetzung von Phenylacetylen mit Methanol und Kohlenmonoxid in Gegenwart eines Oxidationsmittels, wie einer Kombination aus PdCl₂, CuCl₂ und Alkalimetallcarboxylat in Gegenwart eines Katalysators, wie PdCl₂, Pd(NO₃)₂ oder PdSO₄ bekannt.

Aus der BE-A 871 888 ist ein Verfahren zur Herstellung von 2-Alkinsäureestern bekannt, bei dem acetylenische Verbindungen und Dialkylcarbonate in Gegenwart einer starken Base umgesetzt werden. Beispielsweise wird Dimethylcarbonat mit 1-Butin-3-methyl-3-ol in Gegenwart von Natriummethanolat zum Methylester der 4-Methyl-4-hydroxypentinsäure umgesetzt.

Die vorstehenden Verfahren sind teilweise aufwendig in der Durchführung und liefern nicht immer zufriedenstellende Ergebnisse.

Aufgabe der vorliegenden Erfindung ist die Bereitstellung eines Verfahrens zur Herstellung von 2-Alkinsäureestern, das direkt unter sehr milden Bedingungen und mit sehr hohen Ausbeuten zu den gewünschten Produkten führt.

Die Aufgabe wird erfindungsgemäß gelöst durch ein Verfahren zur Herstellung von 2-Alkinsäureestern der allgemeinen Formel (I),

R¹-O-CO-C≡C-R² (I)

in der R¹ und R² unabhängig C₁₋₁₆-Alkyl-, C₆₋₁₂-Aryl, C₇₋₁₂-Aralkyl- oder C₇₋₁₂-Alkarylreste sind und R² auch Wasserstoff sein kann,
durch Umsetzung eines Chlorformiats der allgemeinen Formel (II),

R¹-O-CO-Cl (II)

in der R¹ die vorstehende Bedeutung hat,
mit einem Alkin der allgemeinen Formel (III),

R²-C≡C-H (III)

in der R² die vorstehende Bedeutung hat, in Gegenwart eines Pt- und/oder Pd-Komplexes, der Phosphinliganden und/oder Phosphitliganden aufweist, als Katalysator.

Es wurde gefunden, daß sich 2-Alkinsäureester direkt unter sehr milden Bedingungen und mit sehr hohen Ausbeuten unter Verwendung eines Palladium-Katalysators aus terminalen Alkinen und Chlorformiaten, vorzugsweise in Gegenwart einer stöchiometrischen Menge an Base, darstellen lassen.

Als Katalysatoren werden dabei Pt- und/oder Pd-Komplexe, vorzugsweise Pd-Komplexe eingesetzt, die Phosphinliganden und/oder Phosphitliganden aufweisen. Als Phosphinliganden oder Phosphitliganden sind eine Vielzahl von Liganden geeignet. Beispielsweise können die Liganden die allgemeine Formel PXYZ aufweisen, wobei X, Y und Z unabhängig Alkyl-, Aryl-, Alkoxy- oder Aryloxyreste mit bis zu 18 C-Atomen sind. Bevorzugt sind dabei Alkyl- oder Arylreste, insbesondere Arylreste. Entsprechende Liganden sind beispielsweise in DE-A-1 593 277 beschrieben. Die Pd-Komplexe sind dabei vorzugsweise Pd(0)- und/oder Pd(II)-Komplexe, die organische Phosphin- oder Phosphitliganden, insbesondere Triarylphosphinliganden aufweisen, in denen die Arylreste unabhängig durch C₁₋₆-Alkylreste, vorzugsweise lineare C₁₋₄-Alkylreste, insbesondere lineare C₁₋₃-Alkylreste substituiert sein können. Die Katalysatoren können dabei bei der Umsetzung in situ gebildet werden. Beispiele für Pd(0)-Komplexe sind Tetrakis-(triphenylphosphin)-palladium(0) (Pd(PPh₃)₄) und Tris(dibenzylidenaceton)-dipalladium(0) in Gegenwart von Tri-o-tolylphosphin. Die Komplexe können beispielsweise aus ein- oder zweizähnigen Liganden aufgebaut sein. Beispiele geeigneter Komplexstrukturen sind die folgenden: 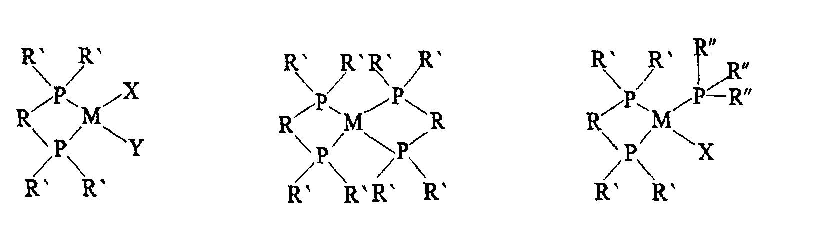 mit der Bedeutung von
- M: Pd, Pt, vorzugsweise Pd
- R: unabhängig an jeder Position organische Reste, die über O- und/-oder C-Atome an die Phosphoratome gebunden sein können, insbesondere Arylreste oder Aryloxyreste mit 2 zur Bindung befähigten Stellen,
- R', R": unabhängig an jeder Position einwertige organische Reste, insbesondere Aryl- und/oder Aryloxyreste
- X, Y: unabhängig einwertige anionische Liganden.

Dabei sind die Ladungen ausgeglichen, wobei gegebenenfalls nicht koordinativ gebundene Kationen oder Anionen zum Ladungsausgleich dienen.

Vorzugsweise leiten sich die einwertigen Reste von Benzol oder Phenol und die zweiwertigen Reste von 1,1'-Biphenyl, 1,1'-Binaphthyl, 1,1'-Biphenyloxyund/oder 1,1'-Binaphthyloxyresten ab. Alle aromatischen Reste können dabei substituiert sein, beispielsweise durch einen oder mehrere C₁₋₆-Alkylreste oder entsprechende Alkoxyreste. Die Biphenyl- und Binaphthylreste, sowie davon abgeleitete Reste sind über 2 Molekülpositionen an das Phosphoratom gebunden. Dabei können die beiden Positionen an dasselbe Phosphoratom gebunden sein. Es ist auch möglich, daß sie an unterschiedliche Phosphoratome gebunden sind und so verbrückte Strukturen entstehen, die beispielsweise 2 Phosphoratome und 3 der genannten Reste aufweisen. Entsprechende geeignete zweizähnige Phosphitliganden sind in US 5,512,695 beschrieben. Die dort beschriebenen Phosphitliganden können auch in analoger Form als Phosphinliganden eingesetzt werden. Weitere geeignete ein- und zweizähnige aromatische Liganden sind in der WO 95/29153 beschrieben. Die beschriebenen Liganden können dabei ebenfalls als Phosphin- oder Phosphitliganden eingesetzt werden. Beispiele verwendbarer Pd(II)-Komplexe sind solche mit ein- oder zweizähnigen Phosphinliganden, wie Pd(PPh₃)₂Cl₂ und Pd(Ph₂P(CH₂)₂PPh₂)Cl₂, die gegebenenfalls in Gegenwart von zusätzlichem Phosphinliganden eingesetzt werden. Ein weiteres Beispiel für einen Pd(II)-Komplex ist ein Gemisch aus Pd(II)acetat und Triphenylphosphin. Die Menge der eingesetzten Pd-Katalysatoren beträgt im allgemeinen 0,01 bis 10 Gew.-%, vorzugsweise 0,5-3 Gew.-%, vorzugsweise 1 bis 2 Gew.-%, bezogen auf die Menge an eingesetztem Alkin. Sie liegen vorzugsweise in homogener Phase im Reaktionsgemisch vor. Die vorstehenden Ausführungen gelten für Pt-Komplexe entsprechend.

Die Reaktionstemperatur beträgt im allgemeinen 0 bis 120°C, vorzugsweise 20 bis 80°C, besonders bevorzugt 30 bis 50°C, insbesondere etwa 40°C.

Dabei kann die Umsetzung lösungsmittelfrei oder in Gegenwart eines inerten Lösungsmittels wie Dichlormethan durchgeführt werden. Die Umsetzung kann bei Normaldruck oder erhöhtem Druck, vorzugsweise einem Druck von 1 bis 20 bar (absolut) durchgeführt werden. Nach Beendigung der Umsetzung kann das Produkt durch Destillation in reiner Form isoliert werden.

Das erfindungsgemäße Verfahren kann kontinuierlich oder diskontinuierlich durchgeführt werden.

Im erfindungsgemäßen Verfahren werden Chlorformiate der allgemeinen Formel (II)

R¹-O-CO-Cl (II)

eingesetzt. Dabei ist R¹ ein C₁₋₁₆-, vorzugsweise C₁₋₆-, insbesondere C₁₋₄-Alkyl-, C₆₋₁₂-Aryl-, vorzugsweise Phenyl-, C₇₋₁₂-Aralkyl-, vorzugsweise C₇₋₁₂-Phenylalkyl oder C₇₋₁₂-Alkaryl-, vorzugsweise C₇₋₁₂-Alkphenylrest. Als Alkylrest kann R¹ linear oder verzweigt sein. Vorzugsweise ist R¹ ein unverzweigter Alkylrest.

Das Chlorformiat der allgemeinen Formel (II) wird mit einem Alkin der allgemeinen Formel (III)

R²-C≡C-H (III)

umgesetzt. Dabei hat R² die gleiche Bedeutung wie vorstehend für R¹ beschrieben. Die Reste R¹ und R² können jedoch unabhängig voneinander ausgewählt werden. Darüber hinaus kann R² auch ein Wasserstoffatom sein. Besonders bevorzugte Reste R² sind n-Butyl, Phenyl oder Wasserstoff.

Die Umsetzung wird vorzugsweise in Gegenwart mindestens einer Base durchgeführt. Dabei wird vorzugsweise eine stöchiometrische Menge mindestens eines sterisch gehinderten Amins eingesetzt. Die sterisch gehinderten Amine sind dabei entweder am Stickstoffatom oder am organischen Rest sterisch anspruchsvoll substituiert. Beispiele sind sterisch anspruchsvoll substituierte Amine, die sich von Piperidin oder Pyridin ableiten. Als Substituenten in den Piperidin- und Pyridinstrukturen kommen dabei C₁₋₁₆-, vorzugsweise C₁₋₄- insbesondere C₁₋₂-Alkylreste, speziell Methylreste in Betracht. Piperidin kann beispielsweise mit 1 bis 7 Methylgruppen, vorzugsweise 3 bis 5 Methylgruppen substituiert sein. Pyridin kann ebenfalls durch Methylgruppen substituiert sein, wobei das Pyridin vorzugsweise mindestens einen C₁₋₆-Alkylaminosubstituenten aufweist. Er ist vorzugsweise in der 4-Position angeordnet. Beispiele geeigneter Basen sind 1,2,2,6,6-Pentamethylpiperidin und 4-Dimethylaminopyridin, die vorzugsweise als Gemisch eingesetzt werden. Dabei beträgt das Molverhältnis von 1,2,2,6,6-Pentamethylpiperidin zu 4-Dimethylaminopyridin vorzugsweise 0,5:1 bis 1:0,5, besonders bevorzugt 0,8:1 bis 1:0,8, insbesondere 0,9:1 bis 1:0,9.

Nachstehend wird die Erfindung anhand von Beispielen näher erläutert.

### Beispiel 1

In einem 500-ml-Rundkolben werden unter Argon 3,25 g *(2,75* mmol) Tetrakis-(triphenylphosphin)-palladium(0) in 250 ml Dichlormethan vorgelegt. Die dunkelbraune Lösung wird 1 h bei Raumtemperatur gerührt. Dann werden 150 mg (1,25 mmol) 4-Dimethylaminopyridin, 21,5 g (137,5 mmol) 1,2,2,6,6-Pentamethylpiperidin und 10,25 g (125 mmol) 1-Hexin zugegeben und unter Rückfluß auf 40°C erwärmt. Anschließend werden innerhalb von 60 min 17 g (125 mmol) n-Butylchlorformiat zugetropft. Nach 24 h Reaktionszeit wird das Reaktionsgemisch destillativ aufgearbeitet, hierbei wird 2-Heptinsäure-n-butylester in 90 % Ausbeute isoliert.

### Beispiel 2

In einem 250-ml-Rundkolben werden unter Argon 1,26 g (1,375 mmol) Tris(dibenzylidenaceton)-dipalladium(0) und 3,34 g (11 mmol) Tri-o-tolylphosphin in 125 ml Dichlormethan vorgelegt. Die rotbraune Lösung wird 1 h bei Raumtemperatur gerührt. Dann werden 75 mg (0,625 mmol) 4-Dimethylaminopyridin, 10,75 g (68,75 mmol) 1,2,2,6,6-Pentamethylpiperidin und 6,38 g (62,5 mmol) Phenylacetylen zugegeben. Es wird unter Rückfluß auf 42°C erwärmt, dann werden innerhalb von 30 min 8,53 g (62,5 mmol) n-Butylchlorformiat zugetropft. Nach 20 h wird das Reaktionsgemisch destillativ aufgearbeitet, hierbei wird Phenylpropinsäure-n-butylester in 98 % Ausbeute isoliert.

### Beispiel 3

In einem 250-ml Rundkolben werden unter Argon 0,25 g (1,1 mmol) Palladium(II)acetat und 1,16 g (4,4 mmol) Triphenylphosphin in 100 ml Dichlormethan vorgelegt. Die gelbe Lösung wird 1 h bei Raumtemperatur gerührt. Dann werden 0,06 g (0,5 mmol) 4-Dimethylaminopyridin, 8,53 g (55,5 mmol) 1,2,2,6,6-Pentamethylpiperidin und 5,1 g (50 mmol) Phenylacetylen zugegeben. Es wird unter Rückfluß auf 40°C erwärmt, dann werden innerhalb von 1 h 6,83 g (50 mmol) n-Butylchlorformiat zugetropft. Nach 20 h Reaktionszeit wird das Reaktionsgemisch destillativ aufgearbeitet, hierbei wird Phenylpropinsäure-n-butylester in 85 % Ausbeute isoliert.

### Beispiel 4

In einem 60 ml-Autoklaven werden unter Argon 0,42 g (0,36 mmol) Tetrakis(triphenylphosphan)-palladium(0), 20 mg (0,16 mmol) 4-Dimethylaminopyridin und 2,87 g (18,5 mmol) 1,2,2,6,6-Pentamethylpiperidin in 30 ml Dichlormethan vorgelegt. Dann werden 10 bar Acetylen aufgepreßt, und der Autoklav wird auf 40°C erwärmt. Innerhalb von 10 min werden 5 g (36,6 mmol) n-Butylchlorformiat zugepumpt, anschließend wird Acetylen auf 20 bar nachgepreßt. Innerhalb von 24 h wird die aufgenommene Acetylenmenge stündlich ergänzt, danach wird abgekühlt, der Reaktionsaustrag mit Stickstoff gespült und das Reaktionsgemisch destilliert. Hierbei wird Propiolsäure-nbutylester in 85 % Ausbeute isoliert.

## Patentansprüche

1. Verfahren zur Herstellung von 2-Alkinsäureestern der allgemeinen Formel (I),
R¹-O-CO-C≡C-R² (I)
in der R¹ und R² unabhängig C₁₋₁₆-Alkyl-, C₆₋₁₂-Aryl, C₇₋₁₂-Aralkyloder C₇₋₁₂-Alkarylreste sind und R² auch Wasserstoff sein kann,
durch Umsetzung eines Chlorformiats der allgemeinen Formel (II),
R¹-O-CO-Cl (II)
in der R¹ die vorstehende Bedeutung hat,
mit einem Alkin der allgemeinen Formel (III),
R²-C≡C-H (III)
in der R² die vorstehende Bedeutung hat, in Gegenwart eines Ptund/oder Pd-Komplexes, der Phosphinliganden und/oder Phosphitliganden aufweist, als Katalysator.

2. Verfahren nach Anspruch 1, **dadurch gekennzeichnet, daß** R² Wasserstoff, ein C₁₋₁₆-Alkyl- oder ein Phenylrest ist.

3. Verfahren nach Anspruch 1 oder 2, **dadurch gekennzeichnet, daß** R¹ ein linearer C₁₋₁₆-Alkylrest ist.

4. Verfahren nach einem der Ansprüche 1 bis 3, **dadurch gekennzeichnet, daß** die Umsetzung in Gegenwart mindestens einer Base durchgeführt wird.

5. Verfahren nach Anspruch 4, **dadurch gekennzeichnet, daß** als Base eine stöchiometrische Menge mindestens eines sterisch gehinderten Amins eingesetzt wird.

6. Verfahren nach einem der Ansprüche 1 bis 5, **dadurch gekennzeichnet, daß** der Pd-Komplex ein Pd(0)- und/oder Pd(II)-Komplex ist, der organische Phosphin- oder Phosphitliganden, insbesondere Triarylphosphinliganden aufweist, in denen die Arylreste unabhängig durch C₁₋₆-Alkylreste substituiert sein können.

7. Verfahren nach einem der Ansprüche 1 bis 6, **dadurch gekennzeichnet, daß** der Katalysator bei der Umsetzung in situ gebildet wird.

8. Verfahren nach einem der Ansprüche 1 bis 7, **dadurch gekennzeichnet, daß** als Katalysator Tetrakis-(triphenylphosphin)-palladium(0) eingesetzt wird.

9. Verwendung von Pt- und/oder Pd-Komplexen, wie sie in einem der Ansprüche 1 bis 3, 6 oder 8 definiert sind, als Katalysatoren zur Herstellung von 2-Alkinsäureestern.

## Claims

1. A process for preparing 2-alkynoic esters of the general formula (I),
R¹-O-CO-C≡C-R² (I)
in which R¹ and R² independently are C₁₋₁₆-alkyl, C₆₋₁₂-aryl, C₇₋₁₂-aralkyl or C₇₋₁₂-alkylaryl radicals, and R² can also be hydrogen,
by reacting a chloroformate of the general formula (II),
R¹-O-CO-Cl (II)
in which R¹ has the above meaning,
with an alkyne of the general formula (III),
R²-C≡C-H (III)
in which R² has the above meaning, in the presence of a Pt and/or Pd complex which has phosphine ligands and/or phosphite ligands as catalyst.

2. A process as claimed in claim 1, wherein R² is hydrogen, a C₁₋₁₆-alkyl or a phenyl radical.

3. A process as claimed in claim 1 or 2, wherein R¹ is a linear C₁₋₁₆-alkyl radical.

4. A process as claimed in any of claims 1 to 3, wherein the reaction is carried out in the presence of at least one base.

5. A process as claimed in claim 4, wherein a stoichiometric amount of at least one sterically hindered amine is employed as base.

6. A process as claimed in any of claims 1 to 5, wherein the Pd complex is a Pd(0) and/or Pd(II) complex having organic phosphine or phosphite ligands, in particular triarylphosphine ligands in which the aryl radicals can independently be substituted by C₁₋₆-alkyl radicals.

7. A process as claimed in any of claims 1 to 6, wherein the catalyst is formed in situ during the reaction.

8. A process as claimed in any of claims 1 to 7, wherein the catalyst employed is tetrakis(triphenylphosphine)palladium(0).

9. The use of Pt and/or Pd complexes as defined in any of claims 1 to 3, 6 or 8 as catalysts for preparing 2-alkynoic esters.

## Revendications

1. Procédé de fabrication d'esters d'acide 2-alcynique de formule générale (I)
R¹-O-CO-C≡C-R² (I)
dans laquelle R¹ et R² représentent, indépendamment l'un de l'autre, un reste alkyle en C₁ à C₁₆, aryle en C₆ à C₁₂, aralkyle en C₇ à C₁₂ ou alcaryle en C₇ à C₁₂ et R² est éventuellement un atome d'hydrogène,
au moyen de la réaction d'un chloroformiate de formule générale (II)
R¹-O-CO-Cl (II)
dans laquelle R¹ prend la signification précédente,
avec un alcyne de formule générale (III)
R²-C≡C-H (III)
dans laquelle R² prend la signification précédente, en présence d'un complexe de Pd et/ou Pt présentant les ligands de phosphine et/ou phosphite, en tant que catalyseur.

2. Procédé selon la revendication 1, **caractérisé en ce que** R² représente un atome d'hydrogène, un reste alkyle en C₁ à C₁₆ ou phényle.

3. Procédé selon la revendication 1 ou 2, **caractérisé en ce que** R¹ représente un reste alkyle en C₁ à C₁₆ linéaire.

4. Procédé selon l'une quelconque des revendications 1 à 3, **caractérisé en ce que** l'on réalise la réaction en présence d'au moins une base.

5. Procédé selon la revendication 4, **caractérisé en ce que** l'on met en oeuvre, en tant que base, une quantité stoechiométrique d'au moins une amine stériquement encombrée.

6. Procédé selon l'une quelconque des revendications 1 à 5, **caractérisé en ce que** le complexe de Pd est un complexe de Pd(O) et/ou de Pd(II) présentant les ligands phosphine et/ou phosphite, en particulier les ligands triarylphosphine, dans lesquels les restes aryle sont, indépendamment les uns des autres, éventuellement substitués par un reste alkyle en C₁ à C₆.

7. Procédé selon l'une quelconque des revendications 1 à 6, **caractérisé en ce que** le catalyseur est formé lors de la réaction *in situ*.

8. Procédé selon l'une quelconque des revendications 1 à 7, **caractérisé en ce que** l'on met en oeuvre, en tant que catalyseur, le tétrakis-(triphénylphosphine)-palladium (0).

9. Mise en oeuvre des complexes de Pt et/ou Pd tels qu'ils sont définis dans l'une quelconque des revendications 1 à 3, 6 ou 8, en tant que catalyseur pour la fabrication d'esters d'acide 2-alcynique.
